(19) Europäisches Patentamt
European Patent Office
Office européen des brevets

(11) Publication number: **0 386 258 A1**

(12) # EUROPEAN PATENT APPLICATION published in accordance with Art. 158(3) EPC

(21) Application number: **89909244.9**

(51) Int. Cl.[5]: **C07D 495/04**

(22) Date of filing: **11.08.89**

(86) International application number: **PCT/JP89/00823**

(87) International publication number: **WO 90/01483 (22.02.90 90/05)**

(30) Priority: **11.08.88 JP 199020/88**

(43) Date of publication of application: **12.09.90 Bulletin 90/37**

(84) Designated Contracting States: **BE CH DE FR GB IT LI SE**

(71) Applicant: **Asahi Kasei Kogyo Kabushiki Kaisha**
**2-6, Dojimahama 1-chome Kita-ku**
**Osaka-shi Osaka 530(JP)**

(72) Inventor: **UEKI, Showa**
**10-13, Hiigawa 5-chome Jonan-ku**
**Fukuoka-shi Fukuoka 813-01(JP)**
Inventor: **KAWAKUBO, Hiromu ASAHI KASEI KOGYO K. K.**
**4100, Asahimachi 6-chome**
**Nobeoka-shi Miyazaki 882(JP)**
Inventor: **OKAZAKI, Katuya ASAHI KASEI KOGYO KABUSHIKI KAISHA**
**4100, Asahimachi 6-chome**
**Nobeoka-shi Miyazaki 882(JP)**
Inventor: **NAGATANI, Tadasi ASAHI KASEI KABUSHIKI KAISHA**
**4100, Asahimachi 6-chome**
**Nobeoka-shi Miyazaki 882(JP)**

(74) Representative: **Boeters, Hans Dietrich, Dr. et al**
**Boeters & Bauer Patentanwälte**
**Bereiteranger 15**
**D-8000 München 90(DE)**

(54) **TETRAHYDROPYRIDINE DERIVATIVES.**

(57) Tetrahydropyridine derivatives represented by general formula (I) (wherein $R_1$ represents an unsubstituted or substituted saturated cycloalkyl group, $R_2$ and $R_3$ each represents halogen, unsubstituted or substituted alkyl, aryl, alkenyl, acyl or arylcarbonyl, and m and n each represents an integer of 0 to 4 provided that $R_2$ groups and $R_3$ groups may be the same or different when m and n are 2 or more) and acid addition salts thereof are disclosed. These compounds are useful as psychotropic drugs acting on central nerves of mammals to exhibit psychoactivating, antianxiety, and learning-improving effects

EP 0 386 258 A1

which have been found by the inventors for the first time.

12.03.90

SPECIFICATION

# TETRAHYDROPYRIDINE DERIVATIVES

## TECHNICAL FIELD

This invention relates to tetrahydropyridine derivatives represented by the following general formula (I) or acid-addition salts thereof which act on the central nervous system of mammals and are useful as psychotropic drugs having psychoanaleptic effects, antianxiety effects and learning improvement effects:

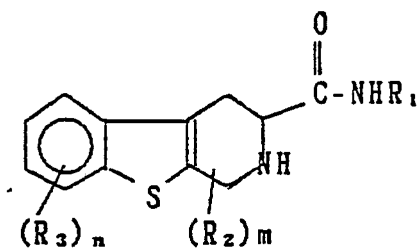

(I)

wherein $R_1$ represents an unsubstituted or substituted saturated cyclic alkyl group; $R_2$ and $R_3$ each represents a halogen atom, an unsubstituted or substituted alkyl group, an unsubstituted or substituted aryl group, an unsubstituted or substituted alkenyl group, an unsubstituted or substituted acyl group or an unsubstituted or substituted arylcarbonyl group; and m and n each represents an integer of from 0 to 4; with the proviso that when m and n are 2 or more, said $R_2$s each may be the same or different.

12.03.90

BACKGROUND ART

It has been known that β-carboline-3-carboxylic acid derivatives represented by the following general formula:

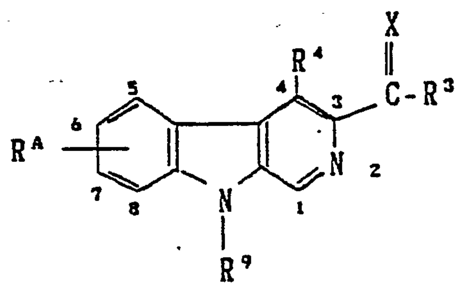

are useful as a psychotic medicine having a sedative activity (U.S. Patent 4,371,536).

Also, it has been known that 1,2,3,4-tetrahydrobenzo[b]-thieno[2,3-c]pyridine derivatives represented by the following general formula:

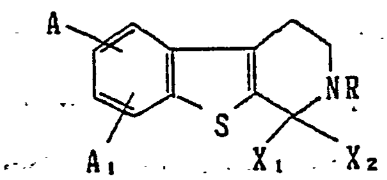

are useful as a central nervous system depressant and an ataraxic (U.S. Patent 3,651,068).

12.03.90

Furthermore, it has been known that pyridine derivatives represented by the following general formula:

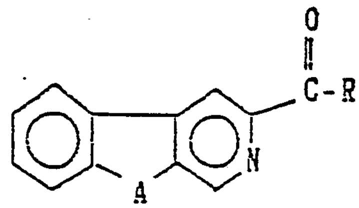

have psychotropic activities (JP-A-61-236779 (The term "JP-A" as used herein means an "unexamined published Japanese patent application")).

Furthermore, studies on the synthesis of 1,2,3,4-tetrahydrobenzo[b]thieno[2,3-c]pyridine or derivatives thereof and biochemical studies of their effect on the brain are described, for example, in Gerhard Wolf and Felix Zymalkowski, Arch. der Pharm., 279, 309 (1976) and in Brandley V. Clineschmidt, Duane R. Reiss, Douglas J. Pettibone and Janet L. Robinson, J. Pharmacol Exp. Ther., 696 - 708, 235 (3) (1985).

Furthermore, WO-88/02751 discloses that compounds represented by the following general formula:

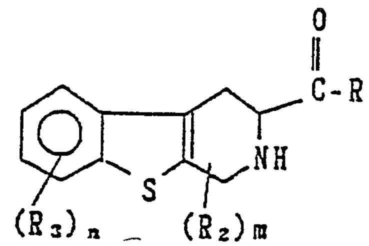

have antianxiety effects and learning improvement effects. It is further disclosed as a general description that R in the above general formula can represent an unsubstituted or substituted amino group.

However, the tetrahydropyridine derivatives represented by the above-mentioned general formula (I) and acid-addition salts thereof have never been known so far and also the medical effects thereof are unknown.

## DISCLOSURE OF INVENTION

The present inventors have conducted extensive studies on methods for preparing tetrahydropyridine derivatives represented by the above general formula (I) and acid-addition salts thereof and usefulness of these compounds in order to provide novel tetrahydropyridine derivatives useful as psychotropic drugs having psychoanaleptic activities, antianxiety activities and learning improvement activities.

The present invention provides tetrahydropyridine derivatives represented by the following general formula (I):

12.03.90

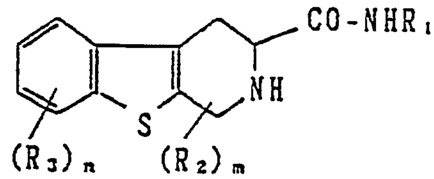

(I)

and acid-addition salts thereof.

In the above general formula (I), $R_1$ represents a saturated cyclic alkyl group, preferably a 3- to 7-membered ring. The hydrogen atoms bonded to carbon atoms in the saturated cyclic alkyl group may be substituted by other substituents. Examples of the substituents include a halogen atom, a carbonyl group, an amino group, an aryl group, an alkyl group having 1 to 6 carbon atoms, an alkenyl group having 1 to 6 carbon atoms and an alkoxy group having 1 to 6 carbon atoms. The hydrogen atoms bonded to carbon atoms and nitrogen atoms in these substituents may be further substituted by other substituents such as an amino group, a carboxyl group or an ester group.

Particular examples of $R_1$ include cyclopropyl, cyclobutyl, cyclopentyl, cyclohexyl and cycloheptyl groups. Among these groups, a cyclohexyl group is preferable.

$R_2$ and $R_3$ each represents a halogen atom, an alkyl group, an aryl group, an alkenyl group, an acyl group or an

arylcarbonyl group, each having about 1 to 20 carbon atoms. The hydrogen atoms bonded to carbon atoms in these groups may be substituted by other groups. Examples of the substituents include a halogen atom, an ester group, an alkoxy group having 1 to 6 carbon atoms, an alkyl group having 1 to 6 carbon atoms and a phenyl group. Examples of $R_2$ and $R_3$ include a chlorine atom, a 2-chlorobenzoyl group, an acetyl group, a 4-methoxybenzoyl group, a 4-aminobutyloyl group, a 4-methoxybenzyl group, a methyl group, an aryl group, a benzyl group and a 4-nitrobenzyl group. m and n each represents an integer of from 0 to 4. When m and n are 2 or more, $R_2$s and $R_3$s each may be the same or different.

Examples of pharmacologically acceptable acid-addition salts include salts of inorganic acids such as hydrochloric, sulfuric and phosphoric acids and those of organic acids such as acetic, p-toluenesulfonic and maleic acids.

The compounds represented by the above-mentioned general formula (I) may be synthesized, for example, by the following method.

In the following reaction scheme, $R_1$ is as defined above and R′ represents an alkyl group having up to 6 carbon atoms or a hydrogen atom, while $X_1$ in $HX_1$, $X_2$ in Boc-$X_2$ and $X_3$ in $HX_3$ each represents a group which becomes an acid by combining with hydrogen atom(s), such as a halogen atom or a methanesulfo group or a group which is excellent as a releasable group such as 4,6-dimethylpyrimidinylmercapto group.

Method:

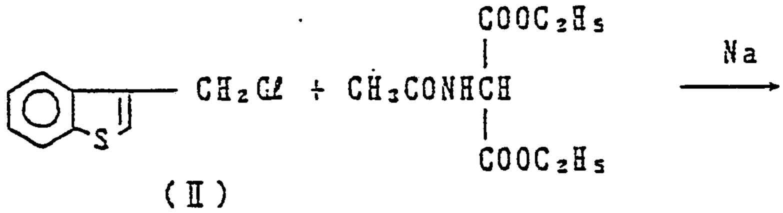


(II)

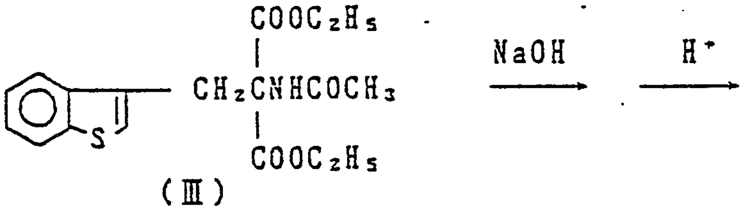


(III)

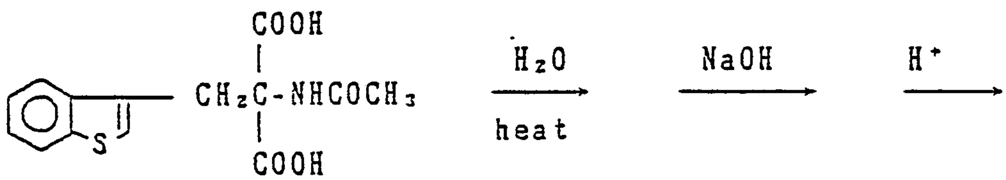


(IV)

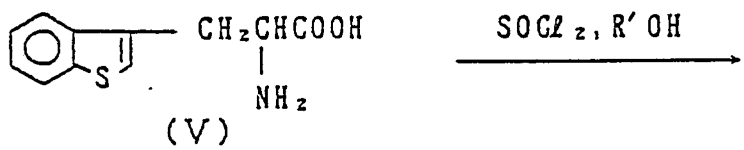


(V)

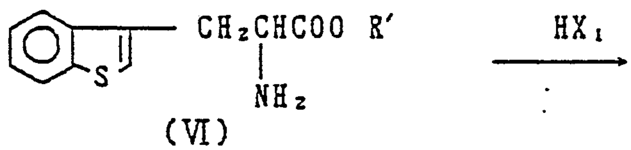


(VI)

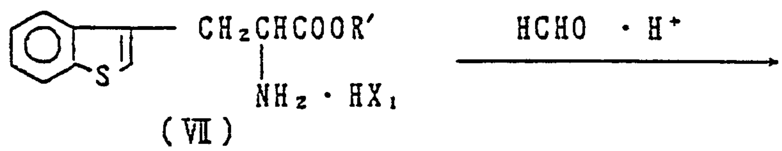


(VII)

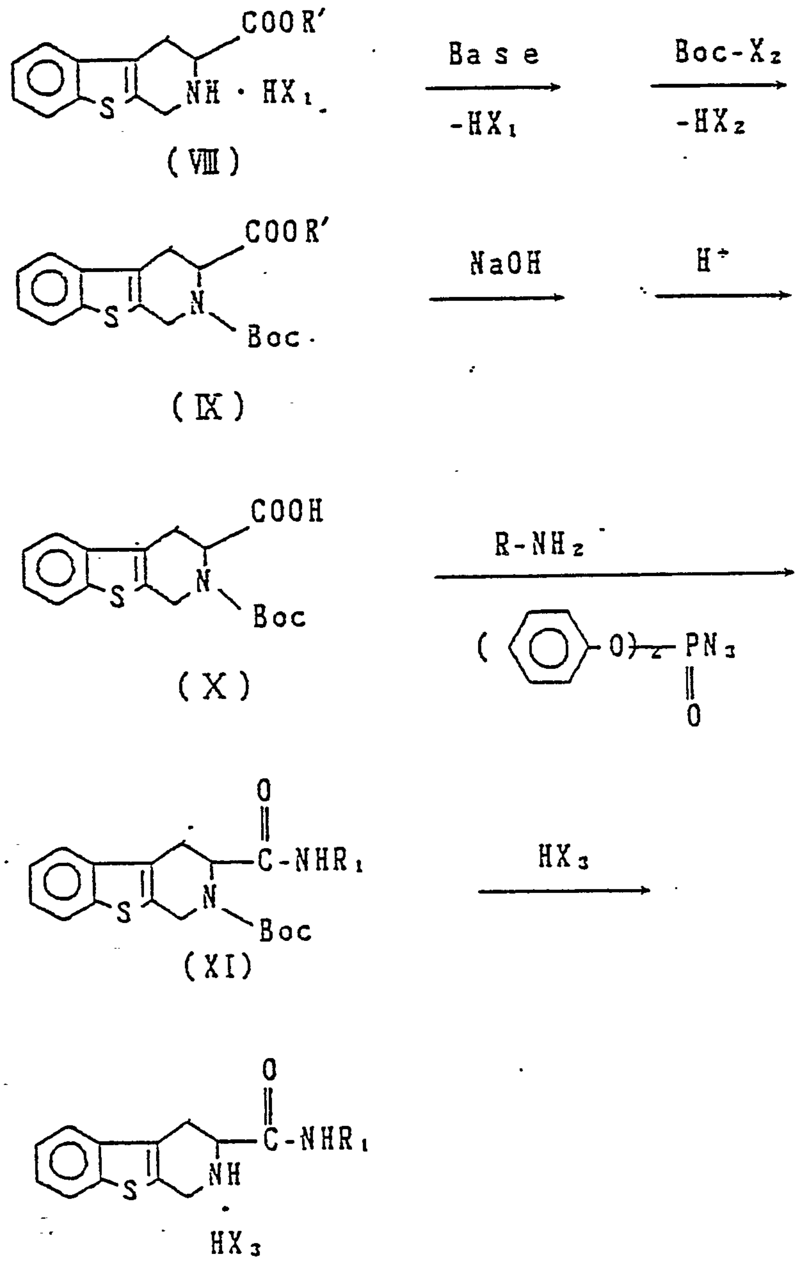


(VIII)

(IX)

(X)

(XI)

The compound (III) is synthesized from the compound (II) according to a method described in Gerhard Wolf and Felix Zymalkowski, Arch. der Pharm., 279, 309 (1976).

Examples of a solvent used in the synthesis of the compound (III) from the compound (II) include ethers such as diethyl ether, tetrahydrofuran and dioxane. This reaction is conducted at from 10 to 120 °C and generally completed within from about 1 to 30 hours. Acetaminomalonic acid diethyl ester is used in an amount of from 1 to 3 equivalents. Metallic sodium may be replaced by an alkali metal such as lithium or potassium.

The compounds (IV) and (V) are obtained from the compound (III) by reference to a method described in H. R. Snyder and Curtis W. Smith, J. Am. Chem. Soc., 66, 350 (1944).

Examples of the solvent to be used in the ester hydrolysis of the compound (III) into the compound (IV) include water, or mixtures of water and lower alcohols such as methanol, ethanol and propanol. Further, sodium hydroxide, potassium hydroxide or sodium carbonate may be used. This reaction is conducted at from 10 to 100 °C and generally completed within from 1 to 10 hours. As an acid for neutralizing the alkali, citric or acetic acid may be used.

The solvent to be used in the decarboxylation and amide hydrolysis of the compound (IV) into the compound (V) is water. The decarboxylation is conducted at from 60 to 100 °C and

generally completed within from about 1 to 30 hours. In the amide hydrolysis, sodium hydroxide may be replaced with potassium hydroxide. This reaction is conducted at from 10 to 100 °C and generally completed within from about 1 to 30 hours.

The compound (VI) is obtained from the compound (V) by reference to a method described in N. Izumiya, T. Kato, M. Ohno and T. Aoyagi, Gosei Kagaku Series (Synthetic Chemistry Series), Peptide Gosei 66 (Peptide Synthesis 66), Example 3-2, ed. by Maruzen. Examples of the alcohols to be used in this method include lower alcohols such as methyl alcohol, ethyl alcohol, propyl alcohol and hexyl alcohol. This alcohol also acts as a solvent. As the acid catalyst, thionyl chloride, sulfuric acid, hydrochloric acid gas or p-toluene-sulfonic acid may be used. This reaction may be conducted at from 10 to 100 °C, preferably from 20 to 30 °C, and generally completed within from 1 to 48 hours.

Examples of the solvent to be used in the reaction for obtaining the compound (VII) from the compound (VI) include lower alcohols such as methyl alcohol, ethyl alcohol, propyl alcohol and hexyl alcohol, and ethyl acetate. An acid-addition salt may be prepared by using, for example, sulfuric acid, hydrochloric acid, or p-toluenesulfonic acid as $HX_1$. This reaction may be conducted at from - 10 to 30 °C, preferably from 0 to 10 °C, and generally completed within a day.

The compound (VIII) is synthesized from the compound (VII) by reference to a method described in D. Soerens et al., J. Org. Chem., 44 (4), 535 - 545 (1979). Formalin is generally used in an amount of from 1 to 10 equivalents, preferably from 1.5 to 2 equivalents, against the compound (VII). The solvent to be used in this method may be a mixture of water with a lower alcohol such as methanol, ethanol, propyl alcohol or hexyl alcohol. This reaction may be conducted at from 10 to 100 °C, preferably from 50 to 70 °C. It may be generally completed within from 1 to 30 hours, preferably from 20 to 25 hours. As the reaction catalyst, an acid such as hydrochloric acid, sulfuric acid or p-toluenesulfonic acid may be used in an amount of 10 to 100 equivalents.

The compound (IX) is obtained from the compound (VIII) by reference to a method described in T. Nakagawa, K. Kuroiwa, K. Narita and Y. Isowa, Bull. Chem. Soc. Japan, 1269, 46 (1973).

Examples of the solvent to be used in the synthesis of the compound (IX) from the compound (VIII) include organic solvents such as chloroform, methylene chloride, tetrahydrofuran and dimethylformamide. This reaction may be conducted at from 0 to 100 °C and generally completed within from 1 to 48 hours. In order to neutralize $HX_1$ in the compound (VIII), a tertiary amine such as triethylamine or N-methylmorpholine is used in an amount of from 2 to 3 equivalents. Further, a butoxy-carbonylating agent such as Boc-azide may be used to

thereby introduce a tertiary butoxycarbonyl group (Boc group). Alternately, the Boc group may be replaced with other amino protecting groups such as a benzyloxycarbonyl group.

The compound (X) is obtained from the compound (IX) by reference to a method described in E. Brand, B.F. Erlanger, H. Sacks and J. Polathick in J. Am. Chem. Soc., 73, 3510 (1951).

The method for synthesizing the compound (X) from the compound (IX) comprises hydrolyzing the starting compound with sodium hydroxide followed by neutralization. The solvent to be used in this reaction may be alcohols such as methanol or ethanol, or water. This reaction may be conducted at from 0 to 80 °C and generally completed within from 1 to 48 hours. The sodium hydroxide is used in an amount of from 1 to 3 equivalents. The sodium hydroxide may be replaced by potassium hydroxide and the like. An acid such as citric acid or acetic acid may be used for neutralizing the alkali.

Examples of the solvent to be used for obtaining the compound (XI) from the compound (X) include aprotic polar solvents such as dimethylformamide or dimethylsulfoxide. This reaction may be conducted at from 10 to 100 °C, preferably from 20 to 30 °C, and generally completed within from 1 to 10 hours.

Examples of the solvent to be used for obtaining an acid-addition salt of the tetrahydropyridine derivative from the compound (XI) include organic solvents such as ethyl acetate. Examples of $HX_3$ include sulfuric acid, hydrochloric acid and p-toluenesulfonic acid. This reaction may be conducted at from 10 to 100 °C, preferably from 40 to 60 °C and generally completed within from 1 to 10 hours. The tetrahydropyridine derivatives may be obtained by neutralizing with a base such as sodium hydroxide, potassium hydroxide or triethylamine.

## BEST MODE FOR CARRYING OUT INVENTION

The present invention is further explained in detail hereinafter by the following examples, but the present invention is not limited to these examples.

### Example 1

First, the synthesis of 2-amino(benzo[b]thiophen-3-yl)-propionic acid ethyl ester hydrochloride will be shown. The following reaction was conducted by reference to a method described in H. R. Snyder and Curtis W. Smith, J. Am. Chem. Soc., 66, 350 (1944).

The starting 3-chloromethylbenzo[b]thiophene was synthesized according to a method described in Arch. der Pharm., 309 (1976), 279 - 288.

5.30 g of metallic sodium was cut into fine pieces and added to 700 ml of dry dioxane. Further, 50 g of acetaminomalonic acid diethyl ester was added at room temperature. The mixture was refluxed under stirring for a day and then 32.3 g of 3-chloromethylbenzo[b]thiophene was added thereto. The mixture was refluxed under stirring for 1.5 day. Then, the reaction mixture was filtered and the filtrate was concentrated

under reduced pressure. The concentrate was purified with a silica gel column and thus 30.5 g of a product was obtained. The NMR and IR data of this product was as follows. Thus, it was identified as ethyl-α-acetamino-α-carbethoxy-β-(3-benzo-[b]thiophene)propionate (yield: 47 %).

IR ($\nu_{max}$, $cm^{-1}$): 3275, 1740, 1640 and 1510.

NMR (δ, $CDCl_3$): 1.30 (t, J = 6 Hz, 6H), 1.95 (s, 3H), 3.67 (s, 2H), 4.17 (q, J = 6Hz, 4H) and 6.50-8.00 (m, 5H).

30.5 g of the ethyl-α-acetamino-α-carbethoxy-β-(3-benzo-[b]thiophene)propionate was added to 250 ml of methanol. To the obtained methanolic solution, was added a solution prepared by dissolving 13.45 g of sodium hydroxide in 500 ml of water. The resulting mixture was refluxed under stirring for 2 hours. Then, the methanol was distilled off under reduced pressure. The aqueous layer was extracted with 300 ml of chloroform twice. The chloroform layer was dried over magnesium sulfate and distilled off under reduced pressure. Thus, 16.75 g of a product was obtained. The NMR and IR data of this product was as follows. Thus, it was identified as α-acetamino-α-carboxy-β-(3-benzo[b]thiophene)propionic acid (yield: 65 %).

IR ($\nu_{max}$, $cm^{-1}$): 1730, 1635 and 1540.

NMR (δ, $CDCl_3$): 1.87 (s, 3H), 3.45 (s, 2H), 7.17-8.00 (m, 5H) and 9.33 (s, 2H).

30 g of the α-acetamino-α-carboxy-β-(3-benzo[b]-thiophene)propionic acid was added to 200 ml of water. The obtained mixture was refluxed under stirring for 3 hours. Then the reaction temperature was lowered to room temperature and 15.6 g of sodium hydroxide was added thereto by portions. The obtained mixture was refluxed under stirring for 2.5 days. After the completion of the reaction, the reaction mixture was cooled to room temperature and washed with 100 ml of chloroform. The pH of the aqueous layer was adjusted to 4.0 with conc. hydrochloric acid. The mixture was allowed to stand in a refrigerator overnight and filtered to thereby give 16.0 g of a product. The NMR and IR data of this product was as follows. Thus, it was identified as 2-amino(benzo[b]thiophen-3-yl)propionic acid (yield: 74 %).

IR ($\nu_{max}$, $cm^{-1}$): 1590, 1420 and 1020.

NMR (δ, $D_2O$, $(CH_3)_3Si(CH_2)_3SO_3Na$): 3.00-4.00 (m, 3H) and 7.20-8.00 (m, 5H).

The following reaction was conducted by reference to a method described in N. Izumiya, T. Kato, M. Ohno and T. Aoyagi, <u>Gosei Kagaku Series, Peptide Gosei 66</u>, Example 3-2, ed. by Maruzen.

920 ml of dry ethanol was cooled to 0 °C and 20.8 ml of thionyl chloride was slowly added thereto. The mixture was stirred at 0 °C for 30 minutes and 16.0 g of 2-amino(benzo-[b]thiophen-3-yl)propionic acid was added at 0 °C, followed by

stirring for 30 minutes. The reaction mixture was stirred at room temperature for 2 days and then the ethanol was distilled off under reduced pressure.

The extraction was carried out by adding 300 ml of methylene chloride and 150 ml of a 5 % aqueous solution of sodium hydrogencarbonate to the residue. The methylene chloride layer was dried over magnesium sulfate and distilled under reduced pressure. Thus 16.9 g of a product was obtained. The NMR and IR data of this product was as follows. Thus, it was identified as 2-amino(benzo[b]thiophen-3-yl)propionic acid ethyl ester (yield: 94 %).

IR ($\nu_{max}$, $cm^{-1}$): 3060, 2950, 1760 and 1590.

NMR ($\delta$, $CDCl_3$): 1.44 (t, J = 5 Hz, 3H), 3.0-3.36 (m, 3H), 4.45 (q, J = 5 Hz, 2H) and 7.18-8.01 (m,5H).

16.9 g of the 2-amino(benzo[b]thiophen-3-yl)propionic acid ethyl ester was dissolved in 50 ml of ethyl acetate. To the obtained solution was added 13.6 ml of 5 N-hydrochloric acid/ethyl acetate. The mixture was allowed to stand at room temperature for 12 hours. The reaction mixture was filtered to thereby give 18.8 g of crystals thus precipitated. The NMR and IR data of this product was as follows. Thus, it was identified as 2-amino(benzo[b]thiophen-3-yl)propionic acid ethyl ester hydrochloride (yield: 97 %).

IR ($\nu_{max}$, $cm^{-1}$): 3070, 2950, 2810, 1760 and 1595.

NMR ($\delta$, $D_2O$, $(CD_3)_2S = O$): 1.45 (t, J = 5 Hz, 3H), 3.01-3.40 (m, 3H), 4.47 (q, J = 5 Hz, 2H), 7.11-8.00 (m, 5H).

N-cyclohexyl-1,2,3,4-tetrahydrobenzo[b]thieno[2,3-c]-pyridine-3-carboamide hydrochloride was obtained by the following method.

23.24 g of the 2-amino(benzo[b]thiophen-3-yl)propionic acid ethyl ester hydrochloride and 10.2 ml of formalin (35 % formaldehyde solution) were dissolved in a mixture of 200 ml of ethanol and 200 ml of water. The obtained mixture was refluxed under stirring for 3 hours. The reaction mixture was concentrated to almost one-half its initial volume and the pH thereof was adjusted to from 9 to 10 with sodium hydrogencarbonate. Then, it was extracted thrice with 300 ml of chloroform. The chloroform layer was washed twice with 100 ml of a saturated aqueous solution of sodium chloride and then dried over magnesium sulfate. Then, the chloroform was distilled off under reduced pressure. The residue was recrystallized from 100 ml of chloroform/ether (1 : 1) to thereby give 14.84 g of a product. The NMR and IR data of this product was as follows. Thus, it was identified as 1,2,3,4-tetrahydrobenzo[b]thieno[2,3-c]pyridine-3-carboxylic acid ethyl ester (yield: 69 %).

IR ($\nu_{max}$, $cm^{-1}$): 2970, 2900, 1720, 1430, 1195, 760 and 740.

NMR (δ, $CDCl_3$): 1.35 (t, J = 6 Hz, 3H), 2.25 (s, 2H), 3.10 (dd, 2H), 3.70-4.00 (m, 1H), 4.30 (q, 2H) and 7.30-8.00 (m, 4H).

6 g of the 1,2,3,4-tetrahydrobenzo[b]thieno[2,3-c]-pyridinecarboxylic acid ethyl ester and 6.63 g of 2-(tertiary-butoxycarbonylthio)-4,6-dimethylpyrimidine were dissolved in 200 ml of dry chloroform and refluxed for 30 minutes. After distilling off the chloroform under reduced pressure, the residue was dissolved in 300 ml of ethyl acetate, washed with 50 ml of a 5 % aqueous solution of sodium hydrogen carbonate thrice, with a 5 % aqueous solution of citric acid twice and with a saturated aqueous solution of sodium chloride twice, and dried over sodium sulfate. The ethyl acetate was distilled off under reduced pressure and the residue was recrystallized from 100 ml of chloroform/petroleum ether (1 : 1) to thereby give 6.18 g of a product. The NMR and IR data of this product was as follows. Thus, it was identified as 2-tert-butoxycarbonyl-1,2,3,4-tetrahydrobenzo[b]thieno[2,3-c]pyridinecarboxylic acid ethyl ester (yield: 76 %).

IR ($\nu_{max}$, $cm^{-1}$): 2970, 1720, 1695, 1400, 760 and 740.

NMR (δ, $CDCl_3$): 1.10 (t, J = 6 Hz, 3H), 1.50 (s, 9H), 3.40 (m, 2H), 4.05 (q, J = 6 Hz, 2H), 4.70 (d, J = 9 Hz, 2H), 5.10-5.50 (m, 1H) and 7.10-7.70 (m, 4H).

6 g of the 2-tert-butoxycarbonyl-1,2,3,4-tetrahydrobenzo[b]thieno[2,3-c]pyridinecarboxylic acid ethyl ester was dissolved in a mixture of 30 ml of methanol and 20 ml of chloroform. 4 ml of a 5N aqueous solution of sodium hydroxide was added thereto and the mixture was refluxed for 5 hours. The solvent was distilled off under reduced pressure, and 300 ml of 5 % citric acid and 300 ml of chloroform were added. The chloroform layer was washed with a saturated aqueous solution of sodium chloride and dried over sodium sulfate. The chloroform layer was distilled off under reduced pressure and the residue was recrystallized from chloroform/hexane to thereby give 4.15 g of a product. The NMR and IR data of this product was as follows. Thus, it was identified as 2-tert-butoxycarbonyl-1,2,3,4-tetrahydrobenzo[b]thieno[2,3-c]pyridinecarboxylic acid (yield: 75 %).

IR ($\nu_{max}$, $cm^{-1}$): 2970, 2860, 1700, 1695, 1400, 760 and 740.

NMR ($\delta$, $CDCl_3$): 1.50 (s, 9H), 3.40 (s, 2H), 4.60 (d, J = 9 Hz, 2H), 5.10-5.50 (m, 1H) and 7.10-7.70 (m, 4H).

12.3 g of the 2-tert-butoxycarbonyl-1,2,3,4-tetrahydrobenzo[b]thieno[2,3-c]pyridinecarboxylic acid was dissolved in 50 ml of dry dimethylformamide. 4.02 g of cyclohexylamine was further added thereto and the mixture was stirred for 30 minutes. A solution of 12.17 g of diphenylphosphoryl azide in

10 ml of dry dimethylformamide and 6.7 ml of triethylamine were further added and the mixture was stirred at room temperature for a day. After distilling off the dimethylformamide under reduced pressure, the residue was dissolved in 300 ml of ethyl acetate, washed with 50 ml of a 5 % aqueous solution of sodium hydrogencarbonate thrice, with a 5 % aqueous solution of citric acid twice and with a saturated aqueous solution of sodium chloride twice, and dried over sodium sulfate. The ethyl acetate was distilled off under reduced pressure and the residue was purified by silica gel column chromatography with the use of chloroform as a development solvent to thereby give 9.17 g of a product. The NMR and IR data of this product was as follows. Thus, it was identified as N-cyclohexyl-2-tert-butoxycarbonyl-1,2,3,4-tetrahydrobenzo[b]thieno[2,3-c]pyridine-3-carboamide (yield: 60 %).

IR ($\nu_{max}$, $cm^{-1}$): 2970, 1720, 1620, 1400, 760 and 740.

NMR ($\delta$, $CCl_4$): 1.47 (m, 19H), 1.47 - 3.10 (m, 1H), 3.16-3.80 (m, 2H), 4.63 (d, J = 16 Hz, 2H), 4.87-5.20 (m, 1H), 6.16 (d, J = 8 Hz, 1H) and 7.00 - 7.83 (m, 4H).

9.17 g of the N-cyclohexyl-2-tert-butoxycarbonyl-1,2,3,4-tetrahydrobenzo[b]thieno[2,3-c]pyridinecarboamide was added to 30 ml of ethyl acetate and 13.3 ml of 5 N-hydrochloric acid/ethyl acetate was further added thereto. After stirring the mixture at 50 °C for 2 hours, N-cyclohexyl-1,2,3,4-tetra-

hydrobenzo[b]thieno[2,3-c]pyridinecarboamide hydrochloride precipitated. After filtering and washing with 30 ml of ether, 6.37 g of a product was obtained. The NMR and IR data of this product was as follows. Thus, it was identified as N-cyclohexyl-1,2,3,4-tetrahydrobenzo[b]thieno[2,3-c]pyridine-carboamide hydrochloride (yield: 82 %).

IR ($\nu_{max}$, $cm^{-1}$): 2935, 1720, 1660, 1430, 750 and 730.

NMR (δ, $d_4$-DMSO): 0.81-2.25 (m, 10H), 2.98-4.35 (m, 5H), 4.54 (s, 2H) and 7.20-8.72 (m, 4H).

Mass(m/e): 314, 188, 172 and 161.

Example 2

The procedure of Example 1 was repeated except that 4.02 g of the cyclohexylamine was replaced by 2.31 g of cyclopropylamine, 2.88 g of cyclobutylamine, 3.45 g of cyclopentylamine and 4.59 g of cycloheptylamine to thereby respectively give N-cyclopropyl-1,2,3,4-tetrahydrobenzo[b]thieno[2,3-c]pyridine-3-carboamide hydrochloride, N-cyclobutyl-1,2,3,4-tetrahydrobenzo[b]thieno[2,3-c]pyridine-3-carboamide hydrochloride, N-cyclopentyl-1,2,3,4-tetrahydrobenzo[b]thieno[2,3-c]pyridine-3-carboamide hydrochloride and N-cycloheptyl-1,2,3,4-tetrahydrobenzo[b]thieno[2,3-c]pyrine-3-carboamide hydrochloride. Table 1-1 shows the results while Table 1-2 shows the analytical data.

Table 1-1

| No. | 2-tert-butoxycarbonyl-1,2,3,4-tetrahydro-benzo[b]thieno[2,3-c]-pyridinecarboxylic acid | Reactant | Reaction temp. | Reaction period | Yield | Product | Final Product |
|---|---|---|---|---|---|---|---|
| 1 | 12.3g | 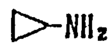 2.31g | Room temp. | 1 day | 9.20g (67%) | 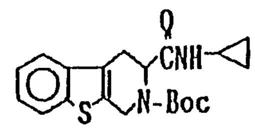 | 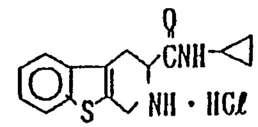 |
| 2 | 12.3g | 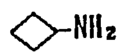 2.88g | do | do | 9.31g (65%) | 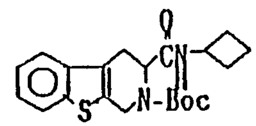 | 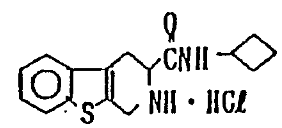 |
| 3 | 12.3g | 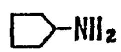 3.45g | do | do | 9.75g (66%) | 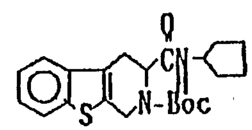 | 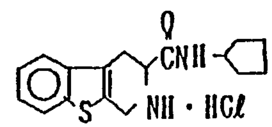 |
| 4 | 12.3g | 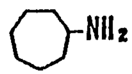 4.59g | do | do | 10.74g (68%) | 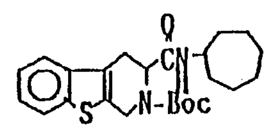 | 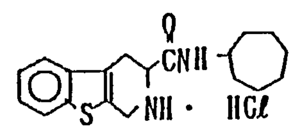 |

Table 1-2

| No. | IR (vmax, $cm^{-1}$) | NMR ($\sigma$, $d_6$-DMSO) | Mass (m/z) |
|---|---|---|---|
| 1 | 2940, 1725, 1655 1430, 750, 735 | 0.41-0.65(m,4H), 2.10-2.46(m,1H) 2.91-4.36(m,5H), 4.55(s,2H), 7.20-8.75(m,4H) | 272, 188, 172, 161 |
| 2 | 2945, 1720, 1655 1435, 740, 725 | 0.85-2.85(m,7H), 2.89-4.26(m,5H), 4.49(s,2H), 7.17-8.73(m,4H) | 286, 188, 172, 161 |
| 3 | 2940, 1718, 1650, 1440, 740, 720 | 0.81-2.55(m,9H), 2.80-4.32(m,5H), 4.40(s,2H), 7.20-8.70(m,4H) | 300, 188, 172, 161 |
| 4 | 2943, 1720, 1645, 1440, 740, 725 | 0.89-2.35(m,13H), 2.85-4.35(m,5H), 4.35(s,2H), 7.20-8.72(m,4H) | 328, 188, 172, 161 |

## INDUSTRIAL APPLICABILITY

(1) Psychoanaleptic Activity:

The psychoanaleptic activities of β-carboline-3-carboxylic acid ethyl ester (β-CCE) which is a typical compound of U.S. Patent 4,371,536; 6-chloro-1,2,3,4-tetrahydrobenzo[b]-thieno[2,3-c]pyridine (C-1) which is a typical compound of U.S. Patent 3,651,068; benzo[b]thieno[2,3-c]pyridine-3-carboxylic acid ethyl ester (A-1) which is a typical compound of JP-A-61-236779; and 1,2,3,4-tetrahydrobenzo[b]thieno[2,3-c]pyridine-3-carboxylic acid ethyl ester (A-2), hexahydro-1-(benzo[b]-thieno[2,3-c]pyridine-3-carbonyl)-1H-1,4-diazepine (A-3), 1-(benzo[b]thieno[2,3-c]pyridine-3-carbonyl)piperidine (A-4), 4-(benzo[b]thieno[2,3-c]pyridine-3-carbonyl)morpholine (A-5), N-(2-aminoethyl)benzo[b]thieno[2,3-c]pyridine-3-carboamide (A-6), N-ethyl-1,2,3,4-tetrahydrobenzo[b]thieno[2,3-c]pyridine-3-carboamide (A-7) and N-hexyl-1,2,3,4-tetrahydrobenzo[b]-thieno[2,3-c]pyridine-3-carboamide (A-8), each a typical compound of WO-88/02751, were compared with the psychoanaleptic activity of N-cyclohexyl-1,2,3,4-tetrahydrobenzo[b]thieno-[2,3-c]pyridine-3-carboamide hydrochloride (A-9), i.e., the compound of the present invention.

This test was conducted in the following manner according to a method described in S. Nomura et al., Eur. J. Pharmacol., 83, 171 - 175 (1982).

Activities of each drug on the akinesia of mice during forced swimming were examined by a mill wheel test. Now the meaning of this test will be described. The akinesia observed during forced swimming of mice may be regarded as emotional disturbance since the animals resign escaping from the narrow environment (device) as the result of learning. It has been known that this akinesia can be shortened by drugs having psychoanaleptic effects such as antidepressants or MAO inhibitors. Namely, each drug was orally administered to a mouse. 40 minutes after the administration, the mouse was introduced into a water tank provided with a mill wheel and contained water at 25 °C. Then, the number of rotations of the wheel was counted throughout the observation period (6 minutes). However, β-CCE was subcutaneously administered to avoid decomposition in digestive organ and the examination was conducted for 15 minutes thereafter (see Margaret M. Schweri, Joseph V. Martin, Wallace B. Mendelson, James E. Barrett, Steven M. Paul and Phil Skolnick, Life Science, 1505-1510, 33 (1983) and P. Skolnick, M. M. Schweri, S. M. Paul, J. V. Martin and W. B. Mendelson, Life Science, 2439-2445, 32 (1983)).

As shown in Table 2, the N-cyclohexyl-1,2,3,4-tetrahydrobenzo[b]thieno[2,3-c]pyridine-3-carboamide hydrochloride (A-9) which is the compound of the present invention, showed a significant increase in the number of rotations of the wheel as compared with other drugs. This result suggests that the N-cyclohexyl-1,2,3,4-tetrahydrobenzo[b]thieno[2,3-c]pyridine-3-carboamide hydrochloride (A-9) according to the present invention has psychoanaleptic activity comparable to those of antidepressants or MAO inhibitors. The results of the test on psychoanaleptic activities as shown in Table 2 indicate that the N-cyclohexyl-1,2,3,4-tetrahydrobenzo[b]thieno[2,3-c]-pyridine-3-carboamide hydrochloride (A-9) according to the present invention is significantly superior to conventional ones and thus seems satisfactorily useful.

Table 2: Psychoanaleptic Activity

| Compound | Dose (mg/kg) | Numbers of Rotations of Wheel |
|---|---|---|
| Control | - | 20.2±7.4 |
| β-CCE | 10 | 23.6±4.1 |
| C-1 | 60 | 24.3±4.3 |
| A-1 | 60 | 23.4±6.8 |
| A-2 | 60 | 26.5±2.9 |
| A-3 | 60 | 22.4±6.7 |
| A-4 | 60 | 24.7±3.9 |
| A-5 | 60 | 21.3±4.0 |
| A-6 | 60 | 25.4±9.1 |
| A-7 | 60 | 19.3±8.1 |
| A-8 | 60 | 20.9±7.6 |
| A-9 | 60 | 37.1±8.0** |

** $P < 0.05$ (t calibration).

Now the antianxiety activities and learning improvement activities of the compounds of the present invention will be shown.

The antianxiety activity and learning improvement activity of the compounds of the present invention were examined by using male Wister rats aged 6 weeks by a water lick conflict test by reference to a method described by Vogel J. R., Beer B. and Clody D. E. described in Psychopharmacologia, 1 - 7, 21 (1971).

In this test, rats having abstained from water were used and an electric shock was applied to the animals every time they drank water to bring the rats into an anxious state. Thus the activity of medicaments to the rats were determined.

(2) Antianxiety Activity:

Rats, having abstained from water for 24 hours prior to the test, were allowed to access to water. After 4 to 5 hours, a drug was administered to the animals. After a definite treatment period, the test was initiated. The shocked number means the number of electric shocks applied to the rats within 5 minutes after initially allowing them to access to water. It shows whether or not the anxiety of receiving electric shock upon drinking the water is restrained. That is, an increase in the shocked number means that the antianxiety activity is increased. Table 3 shows the results where the shocked number of a rat administered with no drug was defined as 100 (n = 5).

For comparison with prior art, the results obtained by the same method as described above by using β-carboline-3-carboxylic acid ethyl ester (β-CCE) which is a typical compound of U.S. Patent 4,371,536; 6-chloro-1,2,3,4-tetrahydrobenzo[b]-thieno[2,3-c]pyridine (C-1) which is a typical compound of U.S. Patent 3,651,068; benzo[b]thieno[2,3-c]pyridine-3-carboxylic acid ethyl ester (A-1) which is a typical compound of JP-A-61-236779; and N-ethyl-1,2,3,4-tetrahydrobenzo[b]thieno[2,3-c]-pyridine-3-carboamide (A-7) and N-hexyl-1,2,3,4-tetrahydro-benzo[b]thieno[2,3-c]pyridine-3-carboamide (A-8) which are typical compounds of WO-88/02751, are also given.

Compared with prior compounds, the compound of the present invention shows an extremely large shocked number, which suggests that the compound of the present invention may be useful as an excellent antianxiety drug.

Table 3: Antianxiety Activity

| Compound | Dose (mg/kg) | Administration Route | Shocked Number |
|---|---|---|---|
| Control | - | - | 100±14.3 |
| N-cyclohexyl-1,2,3,4-tetrahydrobenzo-[b]thieno[2,3-c]-pyridine-3-carbo-amide hydrochloride | 30 | P.O. | 389±41** |
| β-CCE | 40 | P.O. | 82.9±20.0 |
| C-1 | 40 | P.O. | 104.3±28.6 |
| A-1 | 40 | P.O. | 117.1±25.7 |
| A-7 | 40 | P.O. | 127.1±31.5 |
| A-8 | 40 | P.O. | 111.8±28.6 |

** $P < 0.05$ (t calibration).

(3) Learning Improvement Activity:

This test was conducted simultaneously with the measurement of the antianxiety activity (2) and aims at measuring the latent time when the rats abstinence from water first began to drink water.

A longer latent time means the higher learning improvement activity. Table 4 shows the results where the average value of rats administered no drug was defined as 100 ($n = 5$).

12.03.90

Table 4: Learning Improvement Activity

| Compound | Dose (mg/kg) | Administration Route | Latent Time |
|---|---|---|---|
| Control | - | - | 100±16.9 |
| N-cyclohexyl-1,2,3,4-tetrahydrobenzo[b]thieno[2,3-c]pyridine-3-carboamide hydrochloride | 30 | P.O. | 350±32** |
| β-CCE | 40 | P.O. | 312±38.2** |
| C-1 | 40 | P.O. | 128±19.6 |
| A-1 | 40 | P.O. | 132±29.9 |
| A-7 | 40 | P.O. | 232±34.7* |
| A-8 | 40 | P.O. | 249±36.6* |

* $P < 0.1$ (t calibration).

** $P < 0.05$ (t calibration).

Namely, it is suggested that the compound of the present invention significantly prolonged the latent time and thus showed learning improvement activity, which suggests that it may be useful as an excellent antidementia.

(4) Toxicity:

Subacute toxicities of N-(2-aminoethyl)benzo[b]thieno[2,3-c]pyridine-3-carboamide (A-6) and N-cyclohexyl-1,2,3,4-tetrahydrobenzo[b]thieno[2,3-c]pyridine-3-carboamide (A-9) were tested by orally administering these compounds to Wistar rats for 35 days. The test was conducted in the following manner.

Each compound was compulsory orally administered to Wistar SPF rats once a day between 9.00 a.m. to 11:30 a.m. by using a 2 ml or 5 ml disposable syringe and a rat gastric sound for 35 days continuously. Syringes were exchanged for each dose and for each compound. The administration period was determined by referring the initial day of the administration as to 0 day. Table 5 shows the results.

Table 5: Toxicity

| | A-6 | A-9 |
|---|---|---|
| Subeffective Dose (mg/kg) | 50 | 50 |
| Safe Dose (mg/kg) | 50 | 200 |
| Toxic (lethal) Dose (mg/kg) | 200 | 800 |

These results indicate that the toxicity of the compound of the present invention (A-9) is lower than that of the typical compound (A-6) of WO-88/02751.

As described above, the compound of the present invention has psychoanaleptic activity, antianxiety activity and learning improvement activity and yet shows a low toxicity, which indicates that it may be useful as an excellent psychotropic drug.

12.03.90

WHAT IS CLAIMED IS:

Tetrahydropyridine derivatives represented by the following general formula (I) or acid-addition salts thereof:

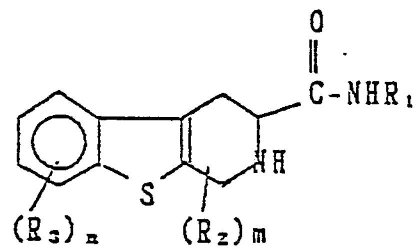

(I)

wherein $R_1$ represents an unsubstituted or substituted saturated cyclic alkyl group; $R_2$ and $R_3$ each represents a halogen atom, an unsubstituted or substituted alkyl group, an unsubstituted or substituted aryl group, an unsubstituted or substituted alkenyl group, an unsubstituted or substituted acyl group or an unsubstituted or substituted arylcarbonyl group; and m and n each represents an integer of from 0 to 4; with the proviso that when m and n are 2 or more, said $R_2$s or $R_3$s each may be the same or different.

# INTERNATIONAL SEARCH REPORT

International Application No PCT/JP89/00823

**I. CLASSIFICATION OF SUBJECT MATTER** (if several classification symbols apply, indicate all) [6]

According to International Patent Classification (IPC) or to both National Classification and IPC

Int. $Cl^4$ C07D495/04

**II. FIELDS SEARCHED**

Minimum Documentation Searched [7]

| Classification System | Classification Symbols |
|---|---|
| IPC | C07D495/04 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, B1, 50-2519 (Colgate-Palmolive Co.)<br>27 January 1975 (27. 01. 75)<br>& US, A, 3518278 & DE, A, 2000775<br>& FR, A, 2035090 & GB, A, 1260971<br>& BE, A, 743692 & DE, A, 1670507 | 1 |
| A | JP, B2, 1-14236 (Pall Call)<br>10 March 1989 (10. 03. 89)<br>& GB, A, 2014576 & BE, A, 874228<br>& EP, A, 3920 | 1 |
| A | JP, A, 61-236779 (Asahi Chemical Industry Co., Ltd.)<br>22 October 1986 (22. 10. 86) (Family : none) | 1 |
| A | JP, A, 63-96188 (Asahi Chemical Industry Co., Ltd.)<br>27 April 1988 (27. 04. 88)<br>& WO, A, 8802751 & EP, A, 285671 | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| August 29, 1989 (29. 08. 89) | September 18, 1989 (18. 09. 89) |
| **International Searching Authority** | **Signature of Authorized Officer** |
| Japanese Patent Office | |

International Application No. PCT/JP89/00823

**FURTHER INFORMATION CONTINUED FROM THE SECOND SHEET**

| | | |
|---|---|---|
| A | JP, A, 63-96189 (Asahi Chemical Industry Co., Ltd.) 27 April 1988 (27. 04. 88) & WO, A, 8802751 & EP, A, 285671 | 1 |

**V. ☐ OBSERVATIONS WHERE CERTAIN CLAIMS WERE FOUND UNSEARCHABLE [1]**

This international search report has not been established in respect of certain claims under Article 17(2) (a) for the following reasons:

1. ☐ Claim numbers ............, because they relate to subject matter not required to be searched by this Authority, namely:

2. ☐ Claim numbers ............, because they relate to parts of the international application that do not comply with the prescribed requirements to such an extent that no meaningful international search can be carried out, specifically:

3. ☐ Claim numbers ............, because they are dependent claims and are not drafted in accordance with the second and third sentences of PCT Rule 6.4(a).

**VI. ☐ OBSERVATIONS WHERE UNITY OF INVENTION IS LACKING [2]**

This International Searching Authority found multiple inventions in this international application as follows:

1. ☐ As all required additional search fees were timely paid by the applicant, this international search report covers all searchable claims of the international application.
2. ☐ As only some of the required additional search fees were timely paid by the applicant, this international search report covers only those claims of the international application for which fees were paid, specifically claims:
3. ☐ No required additional search fees were timely paid by the applicant. Consequently, this international search report is restricted to the invention first mentioned in the claims; it is covered by claim numbers:
4. ☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.